(19) Europäisches Patentamt
European Patent Office
Office européen des brevets

(11) Numéro de publication : **0 161 195**
**B1**

# (12) FASCICULE DE BREVET EUROPÉEN

(45) Date de publication du fascicule du brevet : **19.07.89**

(21) Numéro de dépôt : **85430010.0**

(22) Date de dépôt : **02.04.85**

(51) Int. Cl.[4] : **G 01 N 33/53**, G 01 N 33/531, G 01 N 33/534, G 01 N 33/74, G 01 N 33/577, C 07 D207/46

## (54) Procédé pour le dosage immunologique des monoamines.

(30) Priorité : **10.04.84 FR 8405783**

(43) Date de publication de la demande : **13.11.85 Bulletin 85/46**

(45) Mention de la délivrance du brevet : **19.07.89 Bulletin 89/29**

(84) Etats contractants désignés : **AT BE CH DE FR GB IT LI LU NL SE**

(56) Documents cités :
**EP-A- 0 000 938**
**EP-A- 0 006 792**
**EP-A- 0 095 639**
**FR-A- 2 318 877**
**GB-A- 1 570 818**
**US-A- 3 873 697**
**CHEMICAL ABSTRACTS, vol. 101, no. 1, 2 juillet 1984, page 457, no. 5266e, Columbus, Ohio, US; HARUHISA MITA et al.: "An attempt to produce an antibody to histamine and histamine derivatives" & AGENTS ACTIONS 1984, 14(5-6), 574-9**
**Dépliant pour le dosage de l'Histamine**

**Le dossier contient des informations techniques présentées postérieurement au dépôt de la demande et ne figurant pas dans le présent fascicule.**

(73) Titulaire : **Société Anonyme dite: IMMUNOTECH**
**51 rue Saint Georges**
**F-75009 Paris (FR)**

(72) Inventeur : **Morel, Anne**
**57c rue Marie-Louise**
**F-13008 Marseille (FR)**
Inventeur : **Delaage, Michel**
**16 rue Adolphe Thiers**
**F-13001 Marseille (FR)**

(74) Mandataire : **Rinuy, Santarelli**
**14, avenue de la Grande Armée**
**F-75017 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 161 195 B1

Jouve, 18, rue St-Denis, 75001 Paris, France

## Description

On entend, d'une manière restrictive, par monoamine, une amine résultant de la décarboxylation d'un amino-acide, accompagnée ou non de l'oxydation du résidu. On rencontre de telles amines dans le système nerveux où elles jouent le rôle de neuro-médiateurs. On connaît ainsi :

— les dérivés du tryptophane ou indolamines : tryptamine, 5 hydroxytryptamine (sérotonine), 5 méthoxytryptamine ;

— les dérivés de la tyrosine : tyramine, et catécholamines telles que adrénaline, noradrénaline, dopamine ;

— le dérivé de l'histidine : histamine ;

— le dérivé de l'acide glutamique : l'acide gamma aminobutyrique (GABA).

Du point de vue chimique, le terme monoamine englobe encore bien d'autres molécules, de formule générale $R—NH_2$, où R est un radical hydrocarboné plus ou moins substitué incluant les amino-acides naturels, sans référence particulière au système nerveux.

D'une manière générale, les monoamines sont dosées après chromatographie par une réaction colorée basée sur la fonction amine, telle que la réaction à la ninhydrine, une réaction de fluorescence telle que le couplage avec un résidu dansyl. Plus rarement, on fait appel à une réaction impliquant aussi le radical, comme par exemple la réaction à l'orthophtalaldéhyde par laquelle on dose l'histamine, ou les méthodes polarographiques par lesquelles on dose les indolamines ou les catécholamines (voir « Methods in Enzymology vol. XVII, H. Tabor and C. Tabord Eds, Academic Press N.Y. 1971 » et « Mefford I.N. and Barchas J.D. (1980) J. Chromatogr. 181, 187-193 »).

La caractéristique commune de ces méthodes est le fait qu'elles impliquent au minimum une extraction et une chromatographie qui assurent la spécificité. Leur sensibilité (rarement moins de 10 ng) en limite l'emploi, surtout lorsqu'il s'agit d'analyses biologiques sur des prélèvements nécessairement restreints.

Il faut signaler aussi que, pour les laboratoires capables de préparer des enzymes, on peut utiliser des méthodes radioenzymatiques, basées sur la méthylation des résidus par une méthyl-transférase, par exemple pour l'histamine (C. Bruce, W.H. Taylor and A. Westwood (1979) Annals of Clinical Biochemistry 16, 259-264). Ces méthodes n'ont pas de valeur industrielle.

On a tenté en outre d'appliquer aux monoamines le principe général des immunodosages dans lesquels le composé à doser est mis en compétition avec un analogue radioactif, ou porteur d'un autre type de traceur, pour la fixation à un anticorps capable de les reconnaître l'un et l'autre.

Les molécules de faible masse moléculaire, comme l'histamine ou les autres monoamines, se prêtent mal à cette approche car elles ne contractent pas d'interactions suffisamment fortes avec les anticorps.

L'un des auteurs de la présente invention (M.A. Delaage) a publié une méthode radioimmunologique de dosage de la sérotonine, dans laquelle celle-ci est traitée par l'anhydride succinique en vue d'augmenter l'immunoréactivité de la molécule (J. Physiol. Paris, 1981, 77, 339-347) et une méthode similaire dans laquelle la sérotonine est traitée par l'anhydride acétique (Journal of Neurochemistry, 1982, 39, 1271-1277). Dans chaque cas, le dérivé ainsi obtenu est mis en compétition avec un dérivé radioactif analogue pour la fixation à un anticorps convenable. Toutefois, ces méthodes ne sont pas satisfaisantes car l'apport du chaînon succinate, et a fortiori acétate, ne dépasse pas 1 000 en tant que facteur de gain d'activité, ce qui est insuffisant pour conférer à la molécule une affinité convenable pour son anticorps.

La présente invention remédie à cet inconvénient par le moyen d'une transformation chimique nouvelle qui augmente suffisamment la taille de la molécule pour amener un gain d'affinité de l'ordre de 500 000. Bien entendu, l'anticorps doit être produit contre la molécule modifiée, couplée à une protéine porteuse.

Dans ces conditions, l'immunodosage des monoamines peut atteindre le même niveau de sensibilité et de spécificité que celui des molécules de masse moléculaire plus élevée comme les stéroïdes et les peptides. La procédé selon la présente invention s'applique en particulier aux très petites monoamines de masse moléculaire inférieure à 150, telles que l'histamine. La présente invention a pour objet un procédé de dosage immunologique des molécules présentant une fonction amine primaire ou secondaire dans lequel on procède à la transformation chimique quantitative de ces molécules en dérivés de plus haut poids moléculaire, lesquels sont mis ensuite en compétition avec des analogues radioactifs ou porteurs d'un traceur pour leur fixation à un anticorps capable de les reconnaître les uns et les autres, procédé caractérisé en ce que :

a) Le réactif de transformation chimique des amines en dérivés de plus haut poids moléculaire consiste en un réactif d'acylation (I) de formule générale :

$$R—CO—R' \qquad (I)$$

dans laquelle :

R représente le groupe $—(CH_2)_2—CO—NH—CH_2—CO—NH_2$ et

R' représente un groupement libérable choisi parmi les groupements N- hydroxysuccinimide, les groupes halogène, $—O—CO—R''$ et $—S—R''$ où R'' est un radical hydrocarboné ou le radical $—OC_6H_5$ ;

b) Le traceur possède la formule générale :

$$X—NH—CO—R_1— \quad (II)$$

où $R_1$ est un radical de structure analogue à R couplé soit à une tyrosine radiomarquée soit à une enzyme et X est le reste de la molécule qui porte la fonction amine ;

c) L'anticorps (III) est capable de reconnaître la molécule à fonction amine modifiée et le traceur ; il doit être produit contre cette molécule modifiée couplée à des supports macromoléculaires du type conjugués immunogènes.

La mise en œuvre de ce procédé nécessite la préparation préalable de trois réactifs :

1) Un réactif d'acylation (I) qui réagira avec la fonction amine primaire, la transformant en fonction amide substituée :

$$X—NH_2+R—CO—R' \rightarrow X—NH—CO—R+R'—H$$

monoamine (I) monoamine modifiée (IV)

où X est le support de la monoamine comme par exemple :

X = 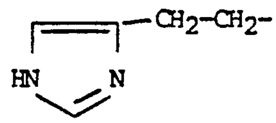 pour l'histamine

X = 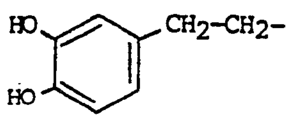 pour la dopamine

Il en serait de même pour les autres amines comme la sérotonine ou autre indolamine, les catécholamines (adrénaline, noradrénaline) ou un amino-acide tel l'acide gamma amino-butyrique, le glutamate et analogues.

(I) est un réactif d'acylation dans lequel :

R représente le groupement $—(CH_2)_2—CO—NH—CH_2—CO—NH_2$.

R' représente un groupement libérable tel que :

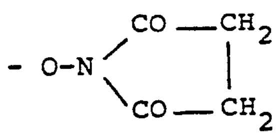 (N-hydroxysuccinimide).

ou —Cl (ou plus généralement halogène)
ou —O—CO—R″ (R″ radical hydrocarboné)
ou —S—R″

ou 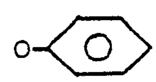

ou tout autre groupement déplaçable par substitution nucléophile.

2) Un traceur radioactif (II), de formule générale :

$$X—NH—CO—R_1—^{125}I$$

où X a la définition ci-dessus, $R_1$ est un radical aussi proche que possible de R, radical apporté à l'amine par le réactif d'acylation.

Suivant une variante avantageuse, le traceur pourra être de type enzymatique : le radical $X—NH—CO—R_1—$ étant alors lié à un résidu lysine d'une enzyme classiquement utilisée à cet effet.

Suivant une autre variante, le traceur sera de type fluorescent.

3) Un anticorps (III) monoclonal ou polyclonal capable de reconnaître la monoamine modifiée (IV), et

le traceur. Un tel anticorps doit être produit contre un conjugué (V ou VI) où le motif (IV) est couplé à une protéine immunogène :

$$X—NH—CO—R_2—CO—NH—\text{albumine} \qquad (V)$$

$R_2$ est un radical lié à R par la relation :

$$—R=—R_2—CO—NH_2,$$

$$R—CO—NH—X—CO—NH—\text{albumine} \qquad (VI)$$

Une fois en possession des trois réactifs ((I), réactif de conversion ; (II), traceur ; (III), anticorps), on procède au dosage de la manière suivante :

1. L'échantillon à doser (quelques centaines de microlitres) est additionné du réactif de conversion, et d'un tampon convenable (neutre ou légèrement alcalin) de sorte que l'ensemble des fonctions amines soit quantitativement transformées en (IV).

2. L'échantillon ainsi transformé est mis en présence du traceur (II) et de l'anticorps en quantités calibrées (III).

3. Après incubation, on procède à la séparation des complexes antigène-anticorps et l'on mesure la radioactivité liée à l'anticorps. Celle-ci sera d'autant plus faible que les molécules X—NH—CO—R (IV) seront plus nombreuses et feront compétition au traceur. La quantité de monoamine $X—NH_2$ présente au départ se déduit par comparaison avec une courbe d'étalonnage.

On procédera de la même manière dans le cadre d'un traceur non radioactif.

Un réactif particulièrement approprié est le composé nouveau répondant à la formule suivante :

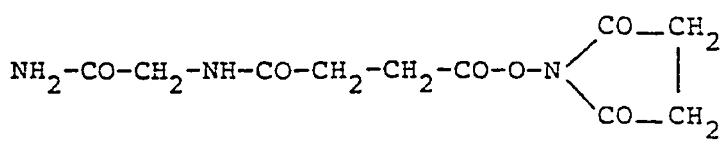

lequel peut être obtenu par succinylation de la glycinamide en faisant réagir un équivalent d'anhydride succinique sur 5 milliéquivalents de glycinamide en présence de 5 milliéquivalents de triéthylamine en diméthylformamide. La succinylglycinamide est séparée à pH neutre, de la glycinamide en excès sur résine échangeuse d'ion de type QAE $A_{25}$ puis éluée à pH acide. Les fractions contenant le produit de couplage sont réunies et lyophilisées.

Les étapes suivantes nécessitent des solvants anhydres (conservés sur tamis moléculaire). 5 milliéquivalents de succinyl glycinamide sont activés 5 mn à 4 °C par 5 milliéquivalents d'éthyl-chloroformiate puis couplés à la N-hydroxysuccinimide en excès (7 milliéquivalents). Le produit de couplage est précipité par addition de dioxane, rincé à l'éther puis lyophilisé.

Le produit se présente sous forme de poudre blanche, soluble dans la diméthylformamide et dans l'eau où il s'hydrolyse rapidement, insoluble dans la plupart des solvants organiques. Son point de fusion est de 108-110 °C. Son poids moléculaire est de 307.7.

L'exemple suivant est donné à titre purement explicatif et nullement limitatif de l'invention :

Histamine transformée par la NOH succinimide ester succinyl glycinamide

1. Le réactif d'acylation (I) est celui précédemment décrit

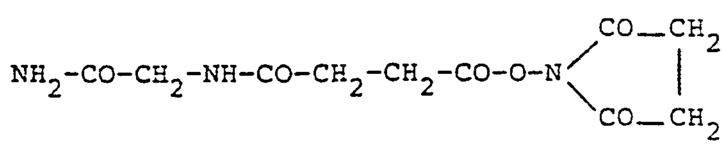

(I)=NOH succinimide ester succinyl glycinamide

Il transforme l'histamine par réaction directe en milieu légèrement alcalin en :

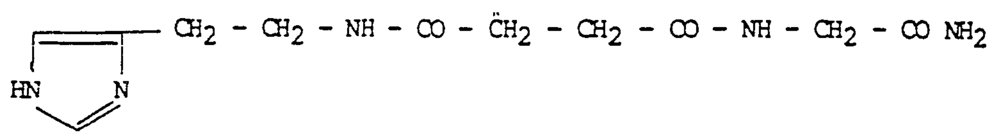

(IV)=histamine succinyl glycinamide

2. Le traceur radiomarqué est obtenu par fixation du succinyl sur la fonction amine puis couplage à la glycyltyrosine qui sera iodée pour donner (II) :

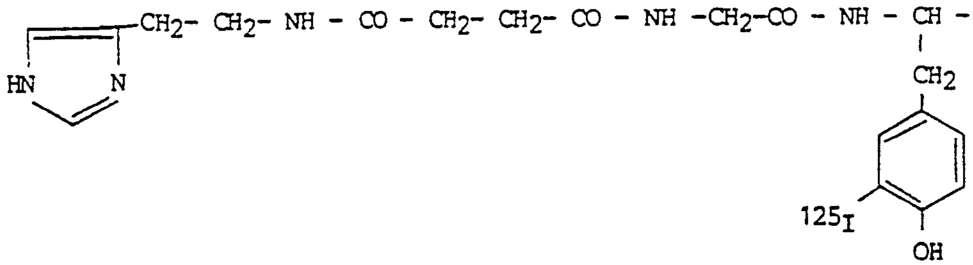

Une variante consiste à accrocher la succinylhistamine à une enzyme à la place de la glycyltyrosine.

3. Les anticorps (III) correspondants sont obtenus par injection chez l'animal du dérivé immunogénique (V) préparé de la façon suivante :

Après fixation du maillon succinyl sur la fonction amine de l'histamine, la succinylhistamine est couplée à la glycylalbumine (de préférence, une protéine non modifiée peut convenir).

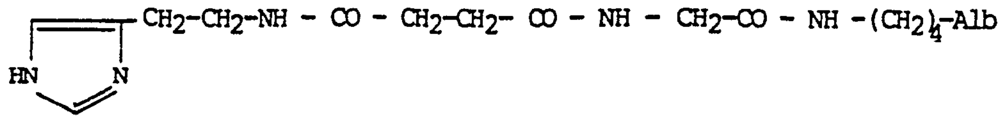

Des anticorps monoclonaux ont été obtenus après injection chez des souris du dérivé immunogénique (V) puis fusion des splénocytes de souris immunes avec des cellules de myélomes de souris (lignée X 63) en présence de PEG 4000 selon la technique décrite par GALFRE G. et al. (Nature, 1977, 266 550-552).

Plusieurs anticorps monoclonaux ont été sélectionnés pour leur capacité à lier le dérivé iodé (II). Le clone 679 AM a été choisi pour son affinité, sa sélectivité pour l'histamine transformée (plus grande discrimination vis-à-vis des analogues de l'histamine). Les anticorps produits en ascite sont purifiés sur protéine A Sépharose® puis fixés sur le tube de dosage selon le procédé décrit dans la demande de brevet français n° 83 05617.

On a porté sur la figure annexée le déplacement du dérivé radiomarqué à l'aide de l'histamine et ses métabolites analogues avant et après transformation de ces molécules. Cette figure illustre le gain de sensibilité par la transformation chimique et la sélectivité du dosage. En effet, après transformation, l'histamine est $5.10^5$ fois mieux reconnue ; de plus, la t-méthyl histamine, produit de dégradation de l'histamine, est $10^4$ fois moins bien reconnue même après transformation. Quant à l'histidine transformée, elle est moins bien reconnue que l'histamine native.

De ce qui précède, l'homme de l'art se rend immédiatement compte qu'il dispose de moyens de dosage immunologique simple des monoamines, ce qui permet de constituer des trousses de dosage renfermant des réactifs prêts à l'emploi mettant en œuvre le procédé de l'invention.

## Revendications

1. Procédé de dosage immunologique des molécules présentant une fonction amine primaire ou secondaire dans lequel on procède à la transformation chimique quantitative de ces molécules en dérivés de plus haut poids moléculaire, lesquels sont mis ensuite en compétition avec des analogues radioactifs ou porteurs d'un traceur pour leur fixation à un anticorps capable de les reconnaître les uns et les autres, procédé caractérisé en ce que :

a) Le réactif de transformation chimique des amines en dérivés de plus haut poids moléculaire consiste en un réactif d'acylation (I) de formule générale :

$$R{-}CO{-}R' \qquad (I)$$

dans laquelle :

R représente le groupement $-(CH_2)_2-CO-NH-CH_2-CO-NH_2$ et

R' représente un groupement libérable choisi parmi les groupements N-hydroxysuccinimide, les groupes halogène, $-O-CO-R''$ et $-S-R''$ où R'' est un radical hydrocarboné ou le radical $-O-C_6H_5$ ;

b) Le traceur possède la formule générale :

$$X{-}NH{-}CO{-}R_1{-} \qquad (II)$$

où $R_1$ est un radical de structure analogue à R couplé soit à une tyrosine radiomarquée soit à une enzyme et X est le reste de la molécule qui porte la fonction amine ;

c) L'anticorps (III) est capable de reconnaître la molécule à fonction amine modifiée et le traceur ; il doit être produit contre cette molécule modifiée couplée à des supports macromoléculaires du type conjugués immunogènes.

2. Procédé de dosage selon la revendication 1 caractérisé en ce que les conjugués immunogènes sont de structure : X—NH—CO—$R_2$—CO—NH—protéine où $R_2$ est lié au réactif (I) par la relation :

$$—R=—R_2—CO—NH_2$$

3. Procédé de dosage selon la revendication 1 ou 2 caractérisé en ce que le réactif (a) est le composé de formule :

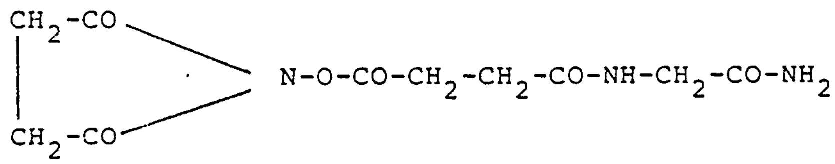

4. Procédé de dosage selon l'une quelconque des revendications 1 à 3 caractérisé en ce que le traceur radioactif résulte de la réaction entre la molécule à fonction amine à doser avec un réactif dérivé de la tyrosine iodoradiomarquée et répond à la formule :

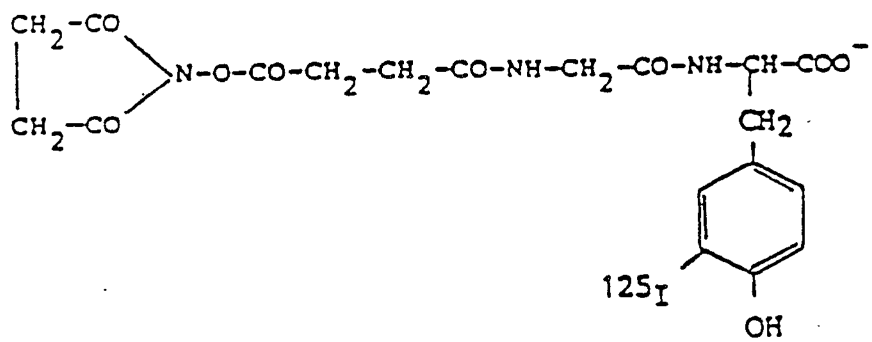

5. Procédé de dosage selon la revendication 4 caractérisé en ce que le motif tyrosine porte une fonction amide ou ester.

6. Procédé de dosage selon l'une quelconque des revendications 1 à 5 caractérisé en ce que la molécule à doser présentant la fonction amine est une monoamine dérivée d'un acide aminé.

7. Procédé de dosage selon la revendication 6 caractérisé en ce que la monoamine est l'histamine.

8. Procédé de dosage de l'histamine selon les revendications 4, 5 et 7 caractérisé en ce que le traceur radioactif est le composé de formule :

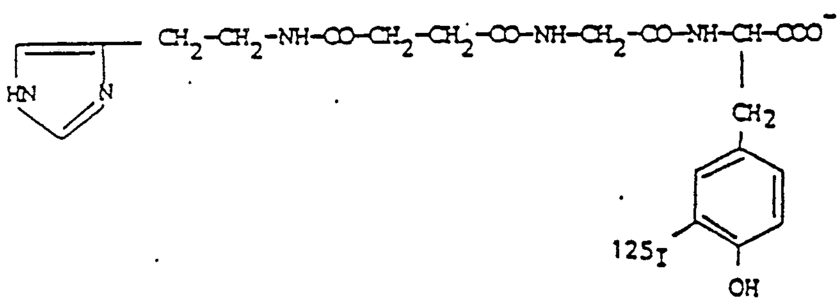

9. Trousse de dosage de l'histamine caractérisée en ce qu'elle comprend les réactifs prêts à l'emploi, à savoir :

(I) le réactif selon la revendication 3

(II) le traceur radioactif selon la revendication 8 ou le traceur enzymatique selon la revendication 1.

(III) l'anticorps capable de reconnaître l'histamine modifiée et le traceur.

10. A titre de produit industriel nouveau le succinylglycinamide N-hydroxy-succinimide ester de formule :

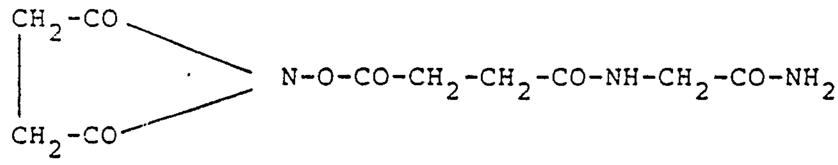

## Claims

1. A method for the immunoassay of molecules having a primary or secondary amine function, in which there is carried out a quantitative chemical transformation of said molecules into derivatives of higher molecular weight, which derivatives are then brought into competition with radioactive analogues or carriers of a tracer in binding to an antibody capable of recognising all of them, which method is characterised in that

a) the reagent for the chemical transformation of the amines into derivatives of higher molecular weight consists of an acylation reagent (I) of the general formula :

$$R—CO—R' \qquad (I)$$

in which

R represents the group $—(CH_2)_2—CO—NH—CH_2—CO—NH_2$ and

R' represents a leaving group selected from N-hydroxy-succinimide, halogen, $—O—CO—R''$ and $—S—R''$, in which R" is a hydrocarbon radical or the radical $—O—C_6H_5$ ;

b) the tracer has the general formula :

$$X—NH—CO—R_1— \qquad (II)$$

in which $R_1$ is a radical of analogous structure to R coupled either to a radiolabelled tyrosine or to an enzyme, and X is the residue of the molecule which of the molecule which has the amine function ;

c) the antibody (III) is capable of recognising the molecule having a modified amine function and the tracer ; and it should be produced against said modified molecule coupled to a macromolecular carrier of immunogenic conjugate type.

2. An assay method according to claim 1, characterised in that the immunogenic conjugates have the structure : $X—NH—CO—R_2—CO—NH—$protein, in which $R_2$ is related to the reagent (I) by the equation :

$$—R=—R_2—CO—NH_2$$

3. An assay method according to claim 1 or 2, characterised in that the reagent (1) is the compound having the formula :

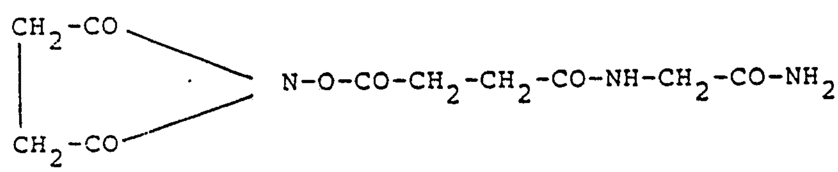

4. An assay method according to any one of claims 1 to 3, characterised in that the radioactive tracer is obtained by reacting the molecule having the amine function to be assayed with a reagent derived from an iodoradiolabelled tyrosine and having the formula :

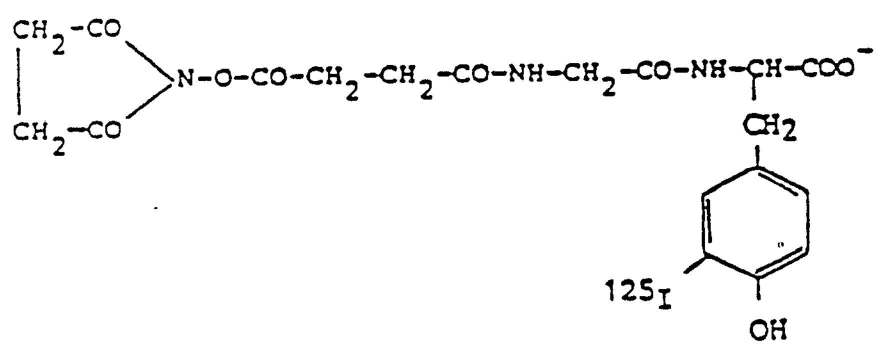

5. An assay method according to claim 4, characterised in that the tyrosine moiety carries an amide or ester function.

6. An assay method according to any one of claims 1 to 5, characterised in that the molecule to be assayed having an amine function is a monoamine derivative of an amino acid.

7. An assay method according to claim 6, characterised in that the monoamine is histamine.

8. A method according to claims 4, 5 and 7 for the assay of histamine, characterised in that the radioactive tracer is a compound having the formula :

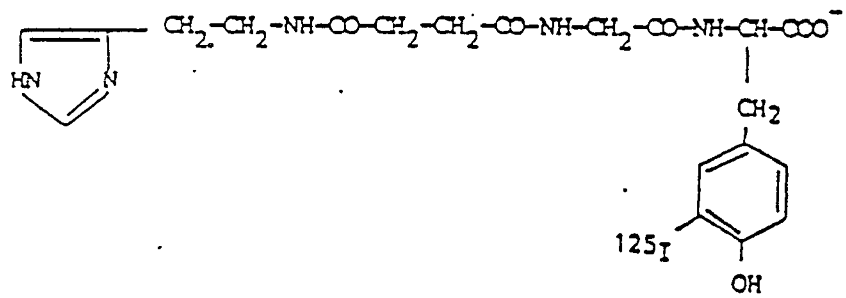

9. A histamine assay kit, characterised in that it comprises ready-to-use reagents, namely :

(I) a reagent in accordance with claim 3 ;

(II) a radioactive tracer in accordance with claim 8 or an enzymatic tracer in accordance with claim 1 ;

(III) the antibody capable of recognising the modified histamine and the tracer.

10. As a novel industrial product the succinyl-glycinamide N-hydroxy-succinimide ester having the formula :

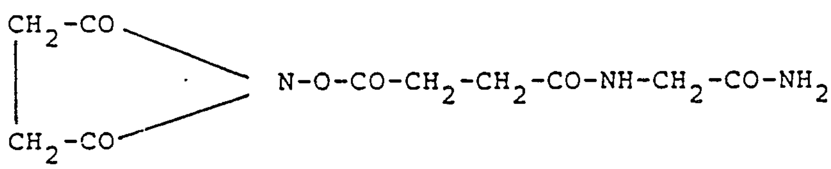

**Patentansprüche**

1. Verfahren zur immunologischen Bestimmung von Molekülen mit einer primären oder sekundären Aminfunktion, in welchem man eine quantitative chemische Umwandlung dieser Moleküle in Derivate mit höherem Molgewicht durchführt, die danach in Konkurrenz mit entsprechenden radioaktiven oder Indikatorträgern an einen Antikörper gebunden werden, der sowohl die einen als auch die anderen erkennen kann, dadurch gekennzeichnet, daß

a) das Reagens zur chemischen Umwandlung von Aminen in Derivate mit höherem Molgewicht aus einem Acylierungsmittel (I) der allgemeinen Formel

$$R—CO—R' \qquad (I),$$

besteht, worin

R die Gruppe $—(CH_2)_2—CO—NH—CH_2—CO—NH_2$ bedeutet und

R' eine abspaltbare Gruppe ausgewählt aus N-Hydroxysuccinimid, Halogen, $—O—CO—R''$ und $—S—R''$ darstellt, wobei R'' einen Kohlenwasserstoffrest oder die Gruppe $—O—C_6H_5$ bedeutet,

b) der Indikator die allgemeine Formel

$$X—NH—CO—R_1— \qquad (II)$$

aufweist, worin $R_1$ ein Rest mit analoger Struktur wie R ist, der entweder an ein radiomarkiertes Tyrosin oder ein Enzym gebunden ist, und X den Rest des Moleküls mit der Aminfunktion darstellt,

c) der Antikörper (III), der das Molekül mit der modifizierten Aminfunktion und den Indikator erkennen kann, gegen dieses modifizierte Molekül, das an makromolekulare Träger vom Typ der immunogenen Konjugate gekoppelt ist, gebildet sein muß.

2. Bestimmungsverfahren nach Anspruch 1, dadurch gekennzeichnet, daß die immunogenen Konjugate die Formel $X—NH—CO—R_2—CO—NH—$Protein aufweisen, worin $R_2$ durch die Relation

$$—R=—R_2—CO—NH_2$$

an das Reagens (I) gebunden ist.

3. Bestimmungsverfahren nach Anspruch 1 oder 2, dadurch gekennzeichnet, daß das Reagens (a) die Verbindung der Formel

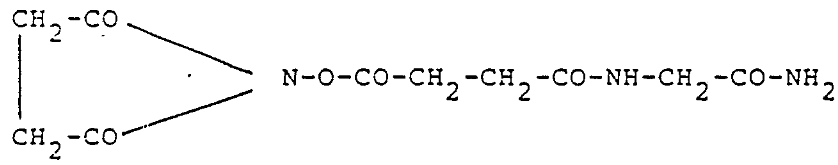

ist.

4. Bestimmungsverfahren nach einem der Ansprüche 1 bis 3, dadurch gekennzeichnet, daß der radioaktive Indikator aus der Reaktion zwischen dem zu bestimmenden Molekül mit Aminfunktion mit einem reaktionsfähigen Derivat von jodradiomarkiertem Tyrosin resultiert und die Formel

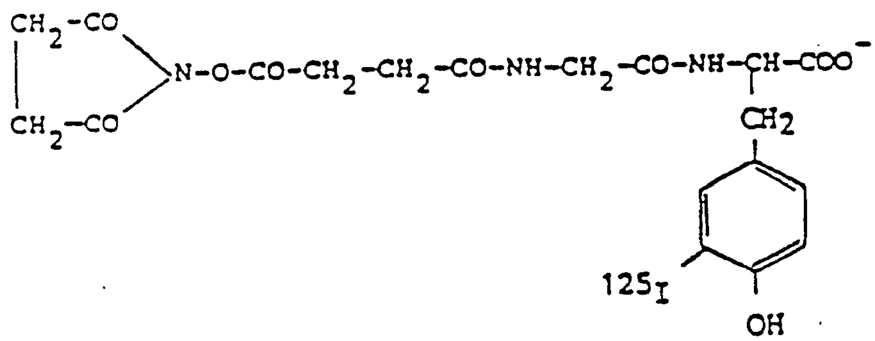

aufweist.

5. Bestimmungsverfahren nach Anspruch 4, dadurch gekennzeichnet, daß die Tyrosingruppe eine Amid- oder Esterfunktion aufweist.

6. Bestimmungsverfahren nach einem der Ansprüche 1 bis 5, dadurch gekennzeichnet, daß das zu bestimmende Molekül mit der Aminfunktion ein Monoaminderivat einer Aminosäure ist.

7. Bestimmungsverfahren nach Anspruch 6, dadurch gekennzeichnet, daß das Monoamin Histamin ist.

8. Verfahren zum Bestimmen von Histamin nach den Ansprüchen 4, 5 und 7, dadurch gekennzeichnet, daß der radioaktive Indikator die Verbindung der Formel

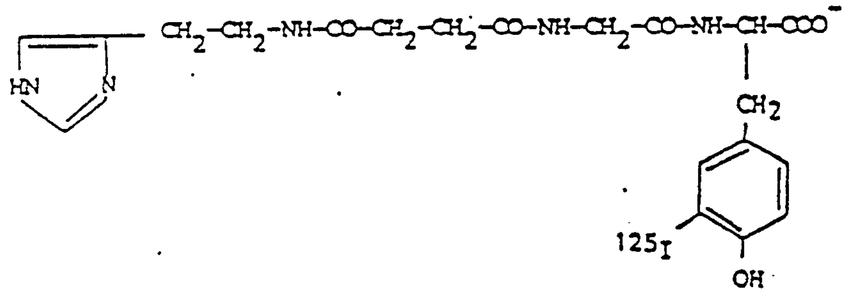

ist.

9. Histaminbestimmungsausrüstung, dadurch gekennzeichnet, daß sie die verwendungsbereiten Reagentien, nämlich

(i) das Reagens nach Anspruch 3,

(ii) den radioaktiven Indikator nach Anspruch 8 oder den enzymatischen Indikator nach Anspruch 1 und

(iii) den Antikörper, der modifiziertes Histamin und den Indikator erkennen kann,

umfaßt.

10. Succinylglycinamid-N-hydroxysuccinimidester der Formel

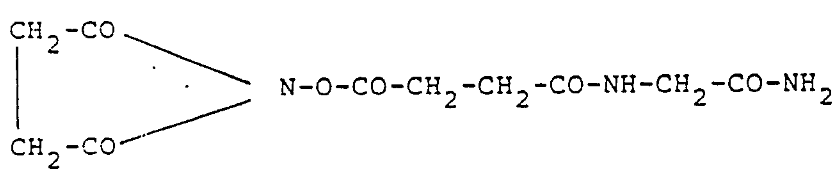

als neues industrielles Produkt.

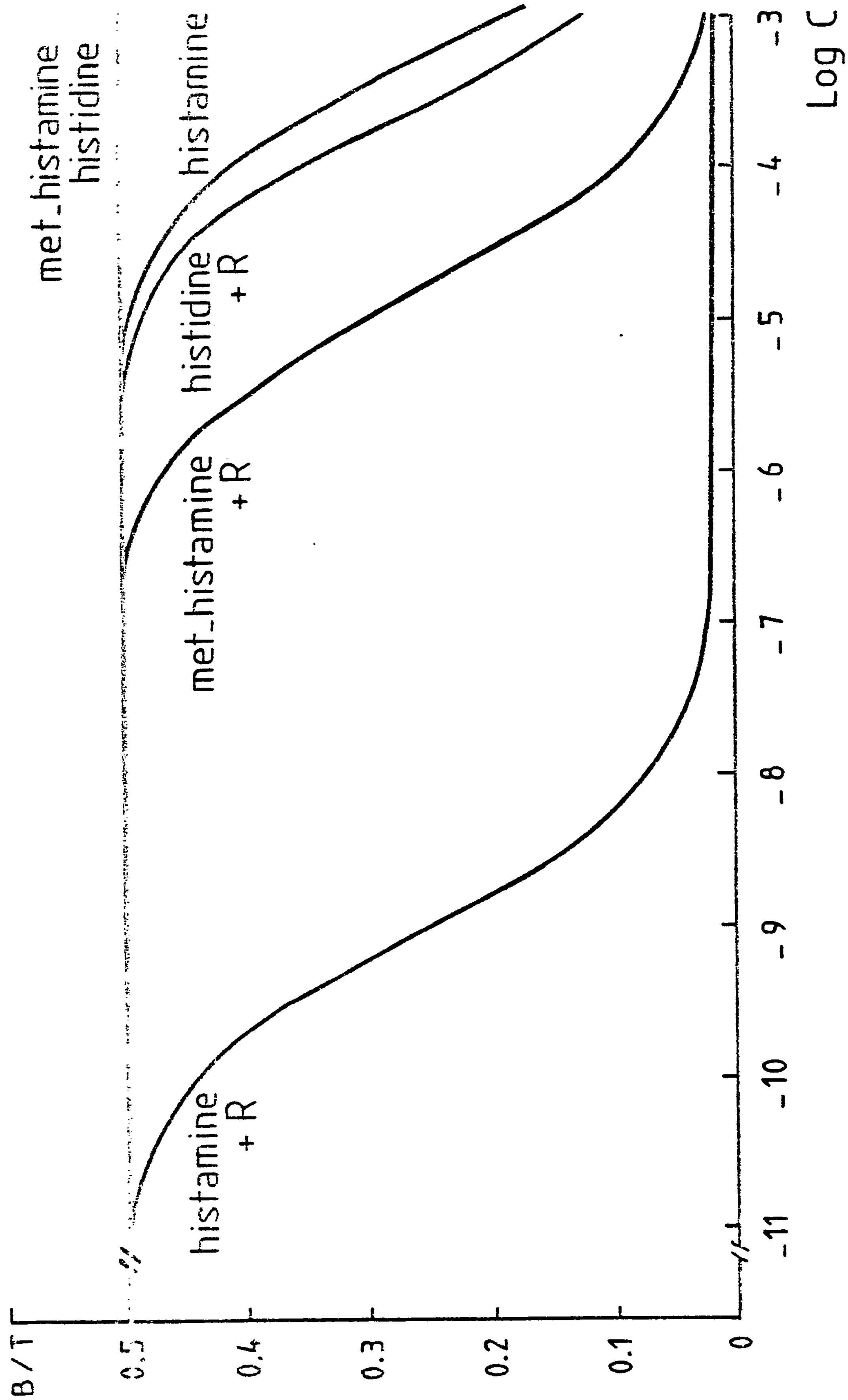
B / T
0.5
0.4
0.3
0.2
0.1
0
Log C
-3
-4
-5
-6
-7
-8
-9
-10
-11
met_histamine
histidine
histamine
histidine
+ R
met_histamine
+ R
histamine
+ R